# EUROPEAN PATENT SPECIFICATION

(11) **EP 4 244 576 B1**
(45) Date of publication and mention of the grant of the patent: **20.08.2025**
(21) Application number: 21806427.7
(22) Date of filing: 11.11.2021
(51) Int. Cl.: G01B 11/06, C03B 9/40, G01B 21/08, G01N 25/72, G01B 21/20

(54) **METHOD FOR INSPECTING HOLLOW GLASS PRODUCTS OF GLASS PRODUCT MATERIAL**
VERFAHREN ZUR INSPEKTION VON HOHLGLASPRODUKTEN AUS GLASPRODUKTMATERIAL
PROCÉDÉ D'INSPECTION DE PRODUITS EN VERRE CREUX EN MATÉRIAU DE PRODUIT EN VERRE

(30) Priority: 11.11.2020 NL 2026865; 14.05.2021 NL 2028216; 14.05.2021 NL 2028215
(43) Date of publication of application: 20.09.2023
(73) Proprietor: Centrum voor Technische Informatica B.V., 9728 JT Groningen (NL)
(72) Inventor: DALSTRA, Joop, 9728 JT Groningen (NL)
(74) Representative: V.O.
(86) International application number: PCT/NL2021/050698
(87) International publication number: WO 2022/103267

(56) References cited:
- EP-A1- 2 743 689
- EP-A1- 3 239 697
- EP-A2- 1 020 703
- EP-A2- 2 333 502
- WO-A1-2019/133504
- DE-B3- 102019 005 487

## Description

The invention relates to a method for inspecting hollow glass products of glass product material, wherein the glass products are manufactured by:
a. heating the glass product material;
b. forming the heated glass product material into at least one glass product in a production flow;
c. cooling the formed glass product;
wherein inspecting the glass products comprises the following steps:
d. transporting the glass products formed in step b. successively along a predetermined path along at least one infrared light sensitive sensor, wherein, of a plurality of the glass products that are transported successively along the at least one sensor, with the at least one sensor per glass product an image is made, wherein step d. is carried out between steps b. and c.;
e. processing the images made in step d. for obtaining information about a wall thickness of the glass products.

The invention further relates to a method for producing and inspecting hollow glass products of glass product material, wherein the glass products are manufactured by:
a. heating the glass product material;
b. forming the heated glass product material into a glass product in a production flow;
c. cooling the formed glass product; wherein inspecting the glass products comprises the following steps:
   d. transporting the glass products formed in step b. successively along a predetermined path along at least one infrared light sensitive sensor, wherein, of a plurality of the glass products that are transported successively along the at least one sensor, with the at least one sensor per glass product an image is made, wherein step d. is carried out between steps b. and c.;
   e. processing the images made in step d. for obtaining information about a wall thickness of the glass products.

Also, the invention relates to a system for producing and inspecting glass products of glass product material according to the above-mentioned method, wherein the system comprises:
a heating apparatus for carrying out step a.;
a product forming apparatus such as a mould for carrying out step b.;
a cooling apparatus for carrying out step c.;
at least one sensor for carrying out step d.; and
a signal processing unit connected with the at least one sensor for processing signals coming from the at least one sensor, the signals each representing an image obtained with the at least one sensor, wherein the signal processing unit is configured for processing the plurality of images for obtaining information about a wall thickness of the glass products.

In addition, the invention relates to a system for inspecting glass products of glass product material according to the above-mentioned method, wherein the system comprises: at least one sensor for carrying out step d.; and
a signal processing unit connected with the at least one sensor for processing signals coming from the at least one sensor, the signals each representing an image obtained with the at least one sensor, wherein the signal processing unit is configured for processing the plurality of images for obtaining information about a wall thickness of the glass products.

Such methods and systems are known per se, for instance from WO-2019133504A1. In the known method, using a plurality of sensors, images of the still-hot just-manufactured glass products are made. Due to the sensors being set up around the glass product, with the images a full revolution of the product is covered. The making of such a group of images is carried out repeatedly at different points of time. On each of the images, the intensity of the infrared radiation is visible. By comparing two images made at different points of time of a same part of the product, a decrease of the intensity can be established. If the intensity decreases relatively slowly, it is established that the glass material at that spot is relatively thick. If the intensity decreases relatively fast, it is established that the glass material at that spot is relatively thin. In this manner, a lateral glass thickness distribution of the glass product can be determined. A disadvantage is that this method is relatively inaccurate.

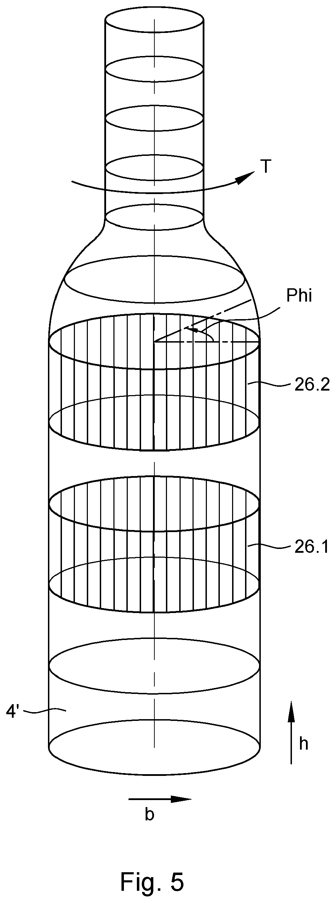

This just-mentioned lateral glass thickness distribution, also referred to by the abbreviation LGD (Lateral Glass Distribution), at a defined height is the set of the wall thicknesses around the circumference of the product (Figure 5). This LGD can be obtained, for instance, by measuring glass wall thicknesses at a defined height h and performing the thickness measurements around the whole circumference of the product. All wall thicknesses of the whole product are the Total Lateral Glass Distribution of the product. A single wall thickness of the product at a defined height h and angle phi (polar coordinates) is an element of the set of wall thicknesses: Lateral Glass Distribution LGD(h,phi). The number of elements in the LGD depends on the, freely to be chosen, measuring resolution of the height and the angle.

The LGD is a very important parameter for the quality of the glass product. The strength of the product is chiefly determined by the thinnest part of a glass wall. In order to prevent breakage in the normal use of the product, the LGD must comply with the specifications of a producer. However, with the current glass production technology, the variation of the Lateral Glass Distribution can range from 35% to 55% of the average glass wall thickness. To arrange for the product to be yet sufficiently strong (minimal reject), this glass thickness variation is compensated for by making the glass wall of extra thick design. As a result, not only does the product become heavier (more glass), but also more base materials are used, it takes more energy to produce the product (melting and annealing) and the transport costs of the glass product become higher due to the extra weight. By minimizing the variation of the lateral glass thickness distribution of the product, the design of the product can be adapted to have a thinner (more constant) glass wall thickness. The product becomes lighter, the production costs fall proportionally, and so do the transport costs (as well as the CO₂, NOₓ emissions depending thereon).

To minimize the variation in the Lateral Glass Distribution in the industrial glass forming process, a sensor that is able to determine the LGD in the glass forming process is requisite. Using this sensor, in the production process the root causes of the variation of the LGD can be investigated, for instance to ascertain which process settings or parts of the process are responsible for an unduly large variation in the LGD. If these causes of the variation of the LGD are known, the responsible process steps can be improved, for instance by optimizing the setting by using the measuring data of the sensor. This may also be done automatically with a feedback system (FeedBack loop) to automate the optimum settings so as to obtain a minimal variation of the LGD. Also, improvements may be incorporated in the responsible process steps, to minimize the variation of the LGD.

Object of the invention is to improve the known inspection process and possibly, on the basis of the improved inspection process, to improve the production process.

The method for inspecting hollow glass products according to the invention is characterised in that the at least one sensor is sensitive to infrared light having at least one frequency where each glass product of the plurality of glass products is transparent to the infrared light so that an image of the plurality of images both shows a side of the glass product that faces the at least one sensor with which the image has been made and shows a side of the glass product, located opposite the side, that faces away from the at least one sensor with which the image has been made, wherein in step d. of each glass product of the plurality of glass products the rotational position of the glass product around an axial axis of the glass product relative to the at least one sensor is determined, wherein the images and the associated rotational positions of the plurality of products are processed in combination for obtaining information about a lateral wall thickness distribution around an axial axis of a virtual glass product that represents the plurality of glass products. Transparent is here understood to mean sufficiently transparent so that an image of the plurality of images both shows a side of the glass product that faces the sensor with which the image has been made and shows a side of the glass product, located opposite the side, that faces away from the sensor with which the image has been made. The sensor can hence see through the product, but does see the inner and outer surfaces of the product. It holds that the sensor is sensitive to light in the spectrum for which the glass is transparent, more particularly that the sensor is sensitive to light having a bandwidth of 900nm - 3500nm, still more particularly that the sensor is sensitive to light having a bandwidth of 900nm - 1900nm.

The invention is based on the first insight that a production fault or production deviation in a glass product will typically occur to a comparable extent and at comparable positions in all successively manufactured glass products. When the glass products have been formed in step b., they all have a same rotational position. A deviation in the forming process of the products is then present at this same position in the successively made glass products. When the glass products are transported along the path, however, the products have mutually acquired a more or less random rotational position. This is a result of the manner in which glass products are transported after step b. is carried out. Accordingly, when a recording of a glass product is made with the at least one sensor, the glass product has a more or less random rotational position with respect to the at least one sensor. This means that mutually different products will have mutually different rotational positions relative to each other and hence relative to the at least one sensor when successively of the products a recording is made with the at least one sensor. A rotational position of a glass product relative to a sensor may for instance be determined from a rotational position of a reference of the product such as a seam or a dot. Each recording thus shows the glass product from a more or less random viewing angle relative to the glass product. By analysing a plurality of recordings in combination with information about the rotational position of the respective products relative to the at least one sensor, an impression can be obtained of what the average product of which the recordings have been made looks like, viewed from different viewing angles relative to that average glass product. This average product is here also called a virtual product. Given sufficient recordings, therefore, a combined recording of the virtual glass product can be obtained that extends around an axial axis of the virtual glass product. And this while only a single sensor needs to be utilized. The result is as if with a plurality of sensors set up in tangential direction of the product around the product, different recordings have been made. The difference is, however, that it is not about a plurality of recordings of a single (same) product, but a recording per product of a plurality of products. The object of the invention is to determine a lateral glass thickness distribution that is representative of the products that are manufactured, this representative lateral glass thickness distribution here being denoted as the lateral glass thickness distribution of the virtual product. A picture of the virtual product need not be determined or be depicted on a display. It is about the lateral glass thickness distribution that is representative of the products manufactured. A virtual product is thus a product having a lateral glass thickness distribution that has been obtained from images of different products that have been made with the at least one sensor, where in the images the rotational positions of the products vary mutually relative to the at least one sensor. The pictures of the real products form in combination a picture that extends (at least partly) around the virtual product.

A virtual product can in fact be regarded as a 3D image of at least a part of a product, where the 3D image has been obtained from a plurality of 2D images of different real products, where these images have been made using the at least sensor and where in the images the rotational positions of the different real products vary mutually relative to the at least one sensor.

Because in general, of different products, images are made where the products have mutually different rotational positions relative to the at least one sensor, from a plurality of such images an image of a virtual product can be obtained that comprises 3D information (and hence information about a lateral glass thickness distribution).

In particular, the image that could be made of the virtual product covers at least a part of a complete product, which part extends completely around an axial axis of the virtual product.

It holds that a virtual product is a product having a lateral glass thickness distribution that has been obtained from images of different real products. The virtual product is a picture of a product that can be obtained by composition from images of different real products, which images upon composition are corrected in respect of the associated rotational positions of the respective products of which the images have been made.

Often, the obtained images of the plurality of products in combination do not cover the complete product. When the product is a bottle, it may happen, for instance, that of the bottom of the bottle no recordings have been made. This is in itself not a problem, because it is not an objective to determine an image of a virtual product, but merely to determine a lateral glass thickness distribution that is representative of the actually manufactured products. The representative glass thickness distribution that is representative of the lateral glass thickness distribution of the actually manufactured products is here called a lateral glass thickness distribution of a virtual product. This because it is not about a lateral glass thickness distribution per manufactured product, but about a lateral glass thickness distribution that has been determined from the images of the different (real) products. In particular, the combining of the mentioned images for obtaining a lateral glass thickness distribution is carried out according to the principle of tomography.

It holds that the lateral glass thickness distribution may be depicted on a display in the form of, for example, a table with numbers. Optionally, the lateral glass thickness distribution may be depicted on a display in an image of a product in which the calculated glass thickness is indicated with numbers or an actual thickness is indicated (visually or with numbers). If solely numerical data of a lateral glass thickness distribution are depicted, this is still about the lateral glass thickness distribution of a virtual product. The view of the virtual product itself, however, is not of interest, only a lateral glass thickness distribution that represents the lateral glass thickness distribution of the actually manufactured products is of interest. If it is desired to represent the determined lateral glass thickness distribution on a display along with a view of the virtual product, this may be done for the virtual product insofar as the images in combination cover a product. If in the images the bottom of a product is lacking, an image of the virtual product may be supplemented on the basis of a specification of the product to be produced. So, if it is desired to depict the virtual product including the calculated lateral glass thickness distribution on a display in an image showing a complete product, then, insofar as the images in combination do not form a complete view of a product, the product specifications may be used to supplement the image to be shown on a display.

Due to the choice of the frequency mentioned, the combined recordings show from different viewpoints both the front and the rear of the glass product. The rear is seen as well, because 'the front of the glass product is seen through'. In this manner, an impression can be obtained of a lateral glass distribution of the glass product.

According to a highly advantageous embodiment, the plurality of images with their associated determined orientations are processed in combination according to the principle of tomography for obtaining the lateral glass thickness distribution of the virtual glass product. With infrared radiation, thus, indirectly the glass thickness is determined. The amount of radiation depends on the temperature (distribution) of the glass and the thickness of the glass (also material properties). In an industrial process the temperature is mostly constant, so that the measurements can be calibrated. The sensor sees a combination of the front glass wall and the rear glass wall. By tomography, this front glass wall and rear glass wall, as well as their surfaces can be respectively distinctly detected. The lateral glass thickness distribution may be expressed in numbers (for instance a glass thickness at a particular position of the glass product) or through an image, for instance an image of a view of the virtual glass product in transparent view where the glass thickness can also be seen. Due to detection of infrared light to which the hollow glass product is transparent, an image of the glass product shows both a side of the glass product that faces the at least one sensor and a side of the glass product, located opposite the side, that faces away from the at least one sensor. However, because the plurality of images are made, which show (at least a part of) the average product from different viewing directions relative to the product, these directions being spread in tangential direction around the virtual product, it is possible to determine according to the principle of tomography the glass thickness of (at least a part of) the wall of the virtual product. This glass thickness can then be determined for at least a first area extending around the virtual glass product. On the basis of the glass thickness distribution it can be determined whether for instance a glass thickness distribution is within predetermined limits. If this is not the case, for instance the glass products of which the recordings have been made for obtaining the lateral glass thickness distribution of the virtual product may be rejected but it is also possible to adjust a parameter of the glass production process, such as for instance the temperature with which the glass product material is heated in step a., or the forming of the heated glass product material into the glass product in step b. In this forming, for instance moulds may be utilized. Adjusting step b. may then for instance consist in replacing a mould with a new mould. Also, in step b. troughs may be utilized through which the glass product material flows in the form of a glass gob towards a mould. Such troughs may, for instance upon an established deviation of a glass thickness distribution, be lubricated with a lubricant. Other adjustments are also possible, of course. These adjustments may then be carried out automatically. It is also possible, however, that some adjustments of step b. are carried out manually.

It holds, preferably, that the glass thickness distribution comprises absolute values of the glass thickness distribution.

It is also possible, however, that the glass thickness distribution solely indicates relative variations in glass thickness. Also, the glass thickness distribution may be shown on a screen by means of a 3D image of a transparent view of the virtual glass product. Here, again, a transparent view of a glass product is understood to be that part of the glass product that is covered by the combination of images. If this combination does not show a complete product, it may, as mentioned, be supplemented with a view of parts of the product according to a predetermined product specification.

Further, it holds, preferably, that each image of the plurality of images both shows a side of the glass product that faces the at least one sensor with which the image has been made and shows a side of the glass product, located opposite the side, that faces away from the at least one sensor with which the image has been made. In this manner, step e. can be carried out particularly accurately.

In particular, it holds that in step e.'s processing in combination of the plurality of images according to the principle of tomography for obtaining the lateral glass thickness distribution of the glass of the virtual glass product, of the at least one sensor solely infrared light is used that relative to a respective sensor comes from the direction of an axial axis of the respective associated glass product of which an image has been made with the respective sensor. In this way too, the accuracy of the information about the lateral wall thickness distribution around the axial axis of a virtual glass product representing the plurality of glass products is improved. In particular, it holds that in steps d. and e. the lateral glass thickness distribution is determined in a first area of the virtual glass product that extends around the axial axis of the virtual glass product. This first area then need not extend throughout the height of the product, but it may. In the first case, it holds preferably that the steps d. and e. are carried out repeatedly for obtaining a lateral glass thickness distribution in a second area of the virtual glass product that extends around the axial axis of the virtual glass product, the first and second area being staggered with respect to each other in the axial direction. The first and second area may partly overlap, adjoin each other, or be apart from each other so that in the latter case between the first and second area is an area that is not covered by the first and second area. Characteristic of these areas is that the lateral glass thickness distribution LGD (h.phi) for the first area can take values of h that cannot be taken in the glass thickness distribution of the second area, since the first and second area are staggered with respect to each other in axial direction.

More particularly, it holds that the steps d. and e. are respectively carried out repeatedly at least three times for respectively obtaining lateral glass thickness distributions in respectively at least three mutually different areas which each extend around the axial axis of the virtual glass product and are staggered with respect to each other in axial direction and which preferably in combination cover at least substantially the whole virtual glass product. In this way, the glass thickness distribution of the whole product can be mapped. Also, as mentioned, it is possible that the first area covers the whole product; in that case the at least one sensor must have a sufficiently large aperture angle in the axial direction of the product.

The method for producing and inspecting hollow glass products is further characterised in that the at least one sensor is sensitive to infrared light having at least one frequency where each glass product of the plurality of glass products is transparent to the infrared light so that an image of the plurality of images both shows a side of the glass product that faces the sensor with which the image has been made and shows a side of the glass product, located opposite the side, that faces away from the at least one sensor with which the image has been made, wherein in step d. of each glass product of the plurality of glass products the rotational position of the glass product around an axial axis of the glass product relative to the at least one sensor is determined, wherein the images and the associated rotational positions of the plurality of products are processed in combination for obtaining information about a lateral glass thickness distribution around an axial axis of a virtual glass product that represents the plurality of glass products. For each method described above, it holds in particular that a sufficiently large number of recordings of products from a production flow are selected in order that these recordings in combination cover the associated virtual product completely around the axial axis of the virtual product. It is possible here, as mentioned, that the combination of images does not cover the product completely in the axial direction. For instance, the bottom may be missing.

The system for producing and inspecting hollow glass products is further characterised in that the at least one sensor is sensitive to infrared light having at least one frequency where each glass product of the plurality of glass products is transparent to the infrared light so that an image of the plurality of images both shows a side of the glass product that faces the at least one sensor with which the image has been made and shows a side of the glass product, located opposite the side, that faces away from the at least one sensor with which the image has been made, wherein the system is configured such that, in use, in step d. of each glass product of the plurality of glass products the rotational position of the glass product around an axial axis of the glass product relative to the at least one sensor is determined, wherein the signal processing unit is configured such that, in use, the images and the associated rotational positions of the plurality of products are processed in combination for obtaining information about a lateral glass thickness distribution around an axial axis of a virtual glass product that represents the plurality of glass products.

The system for inspecting glass products is further characterised in that the at least one sensor is sensitive to infrared light having at least one frequency where each glass product of the plurality of glass products is transparent to the infrared light so that an image of the plurality of images both shows a side of the glass product that faces the sensor with which the image has been made and shows a side of the glass product, located opposite the side, that faces away from the at least one sensor with which the image has been made, wherein the system is configured such that, in use, in step d. of each glass product of the plurality of glass products the rotational position of the glass product around an axial axis of the glass product relative to the at least one sensor is determined, wherein the signal processing unit is configured such that, in use, the images and the associated rotational positions of the plurality of products are processed in combination for obtaining information about a lateral wall thickness distribution around an axial axis of a virtual glass product that represents the plurality of glass products. In particular, it holds for each system described above that the signal processing unit is configured such that, in use, a sufficiently large number of recordings of products from a production flow are selected in order that these recordings in combination cover the associated virtual product completely around the axial axis of the virtual product. It is possible here that the combination of images does not cover the product completely in an axial direction. For instance, the bottom may be missing.

In practice, glass products are typically produced parallel to each other in a plurality of moulds. According to the invention, per mould, for products that have been produced with that mould, the glass thickness distribution of at least one virtual product can then be determined. The glass thickness distribution of a virtual product determined on the basis of products produced with another mould is then determined separately. If one of the moulds exhibits a deviation resulting in a deviation in glass thickness distribution of an associated virtual product, this can be established separately for that mould. Also a deviation in at least one trough each time exclusively supplying a glass gob to one of the moulds, with such deviation resulting in a deviation in the glass thickness distribution of at least one virtual product determined on the basis of products produced from glass gobs which have flowed through the respective at least one trough, can be detected by detection of a deviation in the respective at least one glass thickness distribution. When deviations in troughs and/or moulds have thus been detected, these may be corrected, for instance by readjusting a position and/or orientation of a trough with respect to a mould, and/or by providing a trough with a lubricant, and/or by replacing a mould. This may be carried out automatically or manually. It holds thus for the method in particular that a plurality of the steps b. are carried out parallel to each other for producing parallel to each other a plurality of the products in a plurality of production flows which each comprise a step b., wherein each glass thickness distribution of a virtual product has been obtained on the basis of recordings of products that have been manufactured in a same production flow, more particularly wherein on the basis of at least one determined glass thickness distribution of at least one virtual product that has been obtained from recordings of products stemming from a same production flow, that production flow is controlled (automatically or manually). Controlling of a production flow is here understood to mean controlling of hardware with the aid of which the product is manufactured in the production flow. Such controlling can consist in, for instance, setting a position and/or orientation of at least one trough and/or a mould used in the respective production flow, supplying a lubricant to the at least one trough and/or replacing the respective mould. For the system, it holds in particular that production flows each comprise a step b., wherein the signal processing unit is configured such that, in use, each glass thickness distribution of a virtual product is obtained on the basis of recordings of products that have been manufactured in a same production flow. More particularly, it holds furthermore for the system that on the basis of at least one determined glass thickness distribution of at least one virtual product that has been obtained from recordings of products that stem from a same production flow, that production flow is controlled in an automatic manner.

The invention will now be further explained on the basis of the drawing. In the drawing:
Figure 1 shows a possible embodiment of a system according to the invention for carrying out a method according to the invention;
Figure 2 shows the use of a part of the system of Figure 1;
Figure 3 shows a part of the use according to Figure 2;
Figure 4 shows a possible implementation of the glass product that is manufactured in the system of Figure 1;
Figure 5 shows schematically a 3D view of a glass product in which areas are hatched of which an LGD has been determined;
Figure 6 shows schematically a top plan view of a possible embodiment of the product forming apparatus of Figure 1.

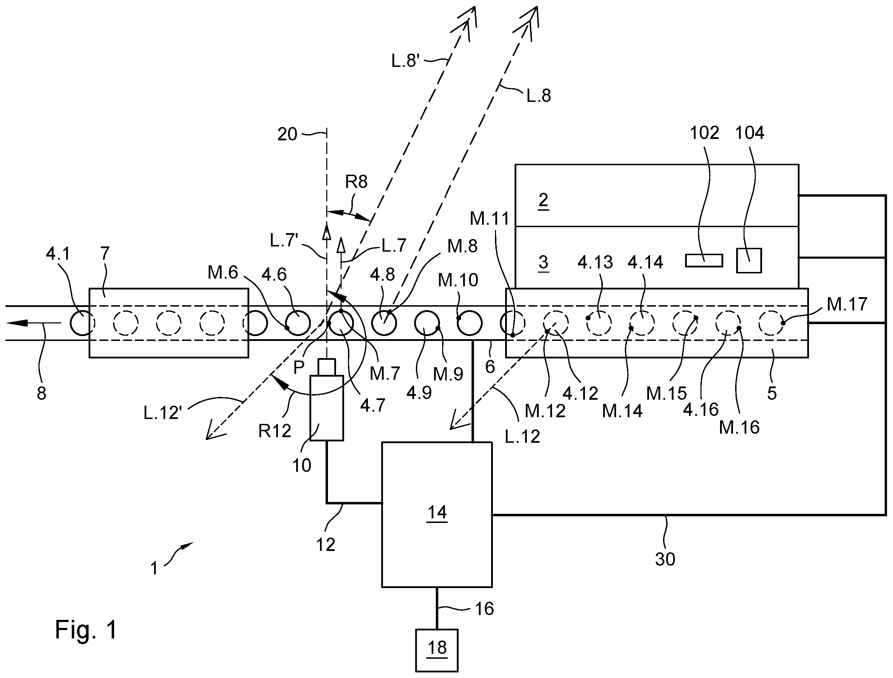

In Figure 1 there is indicated with reference numeral 1 a system according to the invention for carrying out a method according to the invention. The system comprises a schematically shown heating apparatus 2 for heating glass product material so that the glass product material enters a molten state. The molten material is transported to a product forming apparatus 3. In addition, other materials may be supplied to the product forming apparatus 3 if this is necessary, such as other materials and/or semimanufactures. In this embodiment, the product forming apparatus 3 successively manufactures in each case one hollow glass product 4.i (i=1,2,3,...) in a production flow. The glass product 4.i is here manufactured after the glass product 4.i-1. The product forming apparatus 3 in this example includes for this purpose one mould, known per se, 104 (shown schematically in Figure 1) into which a portion of the heated glass product material is introduced, and at least one trough 102 (shown schematically in Figure 1) to guide a glass gob to the mould (the glass gob slides via the inclined trough to the mould concerned). Also, the product forming apparatus 3 comprises blowing means (not shown) for blowing or pushing the glass product material into the mould for obtaining the final shape of the glass product. While in this example the product forming apparatus comprises one mould for manufacturing a glass product in one production flow, it is, of course, also possible that the product forming apparatus comprises a plurality of moulds for manufacturing parallel to each other a plurality of products in a plurality of parallel production flows. An example of this will, after the discussion of the variant with one mould, be discussed as well, with reference to a variant with six moulds.

The successively formed glass products 4.i are placed with the aid of a placing unit 5 on a conveyor 6. For different values of i, then, the corresponding products have been produced at different points of time because products are produced one at a time with one and the same mould in a single production flow.

The glass products 4.i produced as described above are transported with the aid of the conveyor 6 to a position P where inspection of a glass product can take place as will be set out hereinafter. Using the conveyor, the products are then transported further to a cooling apparatus 7 for cooling of the glass product. With the arrow 8, the direction of transport of the conveyor is indicated.

In this example, a sensor, in this example in the form of an infrared camera 10, is arranged for making a recording of a glass product 4.i when the respective glass product 4.i is at the position P. It holds in this example that with the sensor 10 an image is made of a glass product when an axial axis of the respective glass product is at least substantially intersected by an optical axis of the respective sensor. The infrared camera 10 is herein also referred to as (infrared) sensor 10.

Via line 12 which is connected with the infrared camera 10, a signal of the infrared camera is supplied to a signal processing unit 14. The signal processing unit 14 is connected via a line 16 with a display 18.

The working of the system according to the invention is as follows. In a step a., glass product material is heated with the heating unit 2. Then, in a step b., with a product forming apparatus the heated and molten 'liquid' glass material (often having the shape of gob then) is formed into a glass product 4.i. In a step c. the glass product is cooled with a cooling apparatus 7.

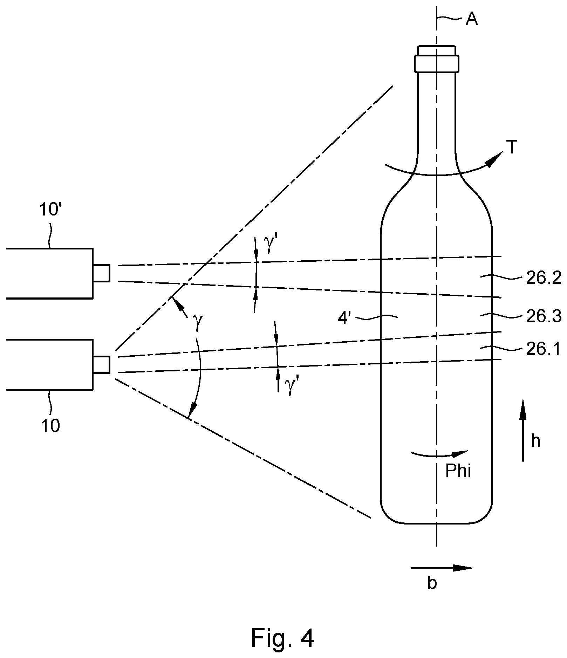

The glass product 4.i formed in the product forming apparatus is placed with the aid of the placing unit 5 on the conveyor 6 for transport in the direction 8. Glass product 4.i, as has been mentioned, is produced after glass product 4.i-1. The products, in this case, are bottles as shown in Figs. 4 and 5. The product 4.i is provided with an axial axis A which in this example is vertically directed. If a product 4.i which has been formed arrives at the position P, a recording of the product is made with the camera 10.

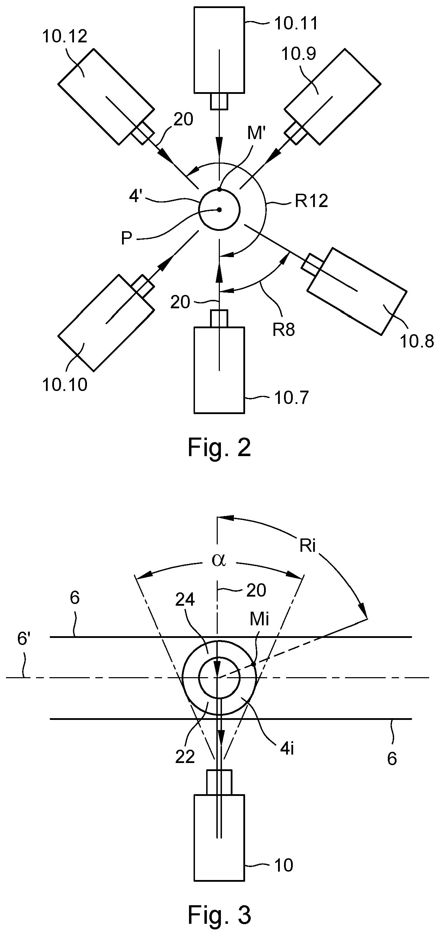

Further, it holds in this example that with the aid of the signal processing unit 14, from the image that is made of the product 4.i with the camera 10, the rotational position R of the glass product on the conveyor around its axial axis relative to, in this example, the viewing direction / optical axis 20 of the camera is determined. This may for instance be carried out by detecting where a marking and/or a seam and/or a dot M of the glass product is. Because a recording thus already comprises information about the rotational position of the glass product that is visible on the recording, it is stated that the rotational position is determined in step d. Because the rotational position is recognized by the signal processing unit from a recording, it may also be said that the rotational position has been determined in step e. The rotational position R may then for instance be an angle R with respect to the viewing direction / optical axis 20 (see Figures 1 and 3). When the glass product is manufactured, it may beforehand be provided with a marking such as a dot or a seam. When the glass product is placed with the aid of the placing means 5 on a conveyor, knowledge about the rotational position of the product is lost because during placing the product can turn about its axial axis.

Thus, therefore, of each product 4.i arriving at the position P an image is made with the camera 10, the information of which is supplied via line 12 to the signal processing unit 14. The signal processing unit determines from the information about the image the rotational position R of the respective product 4.i. Of course, in step d. the rotational position may also be determined in a different way, for instance with a separate sensor whose signals that contain information about the rotational position are supplied to the signal processing unit.

In Figure 1, with M.i (i =.1,2,3,..), respectively, the marking of the glass product 4.i (i = 1,2,3,) is denoted. As can be seen from the markings M.i, the glass products have a random rotational position with respect to the centerline 6' of the conveyor 6 and hence a random rotational position R.i with respect to the viewing direction / optical axis 20. This in contrast to the product forming apparatus 3, in which all products 4.i, as soon as they have been formed, have a same direction of rotation with respect to the centerline 6'. This rotational position, however, can change when a product 4.i is placed on the conveyor.

Due to the products 4.i having a random rotational position Ri on the conveyor 6, the successively made recordings of the products 4.i will show the products in each case from a different viewing direction relative to the marking M.i. If it is assumed that all products are the same, given a sufficient number of recordings, a complete recording in tangential direction of an outer side of the product is available. If the aperture angle of the camera 10 is large enough in vertical direction, the glass product is moreover completely visible in vertical direction. If not, the product is visible in a first area that extends around the product, this area being lower than the height of the product. If there is a fault or a faulty setting present in for instance the glass product forming apparatus, this will typically be present in all glass products 4.i at the same position relative to the marking M.i. By analysing sufficient images of the successively made products 4.i, a fault or deviation can be found in the whole first area (or, if the vertical aperture angle of the camera 10 is sufficiently large, in the whole product). Accordingly, a fully circular image of a virtual product 4' can be constructed from a sufficient number of images of the products 4.i.

This is further clarified in Figure 2. In Figure 2, with camera 10.8 the relative virtual position of the camera 10.8 is indicated with respect to the glass product 4.8 when at the position P an image of the glass product 4.8 is made. Here, in Figure 2, the position and orientation of the glass product 4.8 has been chosen such that M.8 coincides with a randomly fixedly chosen position M' (in this example fixedly chosen to be 'at twelve o'clock' in the drawing). Also, with camera 10.12, the relative virtual position of the camera 10.12 with respect to the glass product 4.12 is indicated, when at the position P an image of the glass product 4.12 is made. Here, in Figure 2, the relative position of the product 4.12 has again been chosen such that M.12 coincides with the randomly fixedly chosen position M'. In Figure 2, thus, the rotational position of the glass product 4.8 is imaged so as to be coincidental with the rotational position of the product 4.12, such that M.8 coincides with M.12. Looking at the real position of the camera 10 with respect to the glass product 4.12 when the glass product 4.12 is at the position P, the viewing direction/optical axis 20 of the camera 10 and the rotational position of the product 4.12, measured with respect to the marking M.12, include an angle R12 (see Figure 1). The rotational position of the product 4.12 is indicated in Figure 1 with the line L12. The line L12' is parallel to the line L12 and indicates the rotational position of the product 4.12 when it would be at the position P. In that position, the rotational position of the product 4.12 with respect to the viewing direction/optical axis 20 of the camera can be indicated with the angle R12. In Figure 2 the same rotational position R12 is indicated. However, because in Figure 2 the rotational position of the product 4.12 is imaged so as to be coincidental with the rotational position of the product 4.8 (since M.8 and M.12 coincide in Figure 2), the virtual position of the camera 10.12 will not coincide with the virtual position of the camera 10.8. In Figure 2, entirely analogously, for the products 4.7, 4.9, 4.10 and 4.11, the relative positions of the associated cameras 10.7, 10.9, 10.10 and 10.11 are indicated. The images of the virtual cameras 10-7 - 10-12 in Figure 2 form in combination an image extending around a virtual product 4'. To put it differently, the images of the virtual cameras 10.i are equivalent to images of real cameras which are set up at the positions of virtual cameras in Figure 2 around a real product which is set up at the position of the virtual product in Figure 2. It follows, therefore, that a measurement with the virtual sensor 10.i on the virtual product 4' in Figure 2 is equivalent to a measurement with the real sensor 10 on product 4.i in Figure 1.

In Figure 2 the position of the virtual camera 10.12 has therefore been chosen such that it has the rotational position R.12 with the virtual product 4' with marking M'. To put it differently: in Figure 2 the product 4.12 and the camera 10 have been rotated around the axial axis of the product 4.12 at the position P such that the marking M.12 of the product 4.12 coincides with the marking M.8 of the product 4.8 (which already coincided with M' by the above-mentioned rotation of the camera 10 to the virtual camera 10.8), wherein after rotation the position of the camera 10 for product 4.12 is indicated with 10.12 and wherein after rotation the position of the camera 10 for product 4.8 is indicated with 10.8. This has been done in Figure 2 entirely analogously for the products 4.7, 4.9, 4.10 and 4.11. For instance, entirely analogously, in Figure 1, the rotational position of the product 4.11 is indicated with the line L11. The line L11' is parallel to the line L11 and indicates the rotational position R.11 = 0 of the product 4.11 when it would be at the position P. In that position, the rotational position of the product 4.11 with respect to the viewing direction/optical axis 20 of the camera 10 can be indicated with the angle R11. In Figure 3 the rotational position R.i for the random product 4.i is indicated.

In this example, it holds that when in each case six images are combined, the virtual product is visible from all sides in the set of images. Of course, it may also be chosen to combine eight images in each case. A first set of eight images that are combined may or may not overlap with a next set of images that are combined. Also, it is conceivable that in each case a variable sufficient number of images are combined that show the virtual product that can be formed from these images as being visible from all sides (that is, the images in combination cover the virtual product completely in circular tangential direction).

In this example, the camera has an aperture angle in a horizontal plane so that an image that is made with a camera covers the whole product in horizontal direction. This is not requisite, however. In Figure 3 the aperture angle concerned is indicated with α. However, the aperture angle α may also be larger than or smaller than indicated. Viewed in vertical direction, the aperture angle y (contained in a vertical plane) of the camera is such that likewise the whole product 4.i is covered (see Figure 4). However, the angle y may also be smaller than or larger than indicated.

It will be clear that the making of the plurality of images in step d. is carried out between the steps b. and c.

Each image that is made with an infrared camera 10 is respectively supplied via line 12 to the signal processing unit 14. These signals are processed in combination in a step e., for obtaining at least one parameter that depends on a wall thickness of the glass product.

As mentioned, the infrared camera applied in step d. is sensitive to infrared light. In particular, the camera 10 is sensitive to infrared light having at least one frequency where the glass product is transparent to the infrared light. Transparent is here understood to mean sufficiently transparent so that an image of the plurality of images both shows a side of the glass product that faces the sensor with which the image has been made and shows a side of the glass product, located opposite the side, that faces away from the sensor with which the image has been made. The camera can thus see through the product, but does see the inner and outer surfaces of the product. This has as a result that in an image that is made with a camera 10, both a side 22 of the glass product that faces the sensor (see Figure 3) is visible and the side 24, located opposite the side 22, that faces away from the infrared camera 10. For the side 24 is visible to the camera 10 in Figure 3 because the camera 10 can also "see through the side 22". Having regard to the aperture angle α, the image obtained with the aid of the camera 10 covers the whole product in the direction Phi (see Figure 4), but it also holds, of course, that the plurality of images in combination covers a first area of the virtual glass product 4' that extends in tangential direction around an axial axis of the product. This first area, in this example, also covers the whole product seen in vertical direction. The images thus overlap each other in tangential direction T (see Figure 4) of the product so that different images show a same part of the glass product. In this case, the camera 10 covers the complete product 4.i, both in the axial direction (vertical direction) and in the width direction b (horizontal direction) of the product. In this example, the set of six images are processed in combination for obtaining an image of the virtual product 4'. It holds then, accordingly, that in step d. of each glass product of the plurality of glass products the rotational position of the glass product around an axial axis of the glass product relative to the at least one sensor is determined, wherein the images and the associated rotational positions of the plurality of products are processed in combination by the signal processor 14 for obtaining information about a lateral wall thickness distribution around an axial axis of a virtual glass product that represents the plurality of glass products. For, since in the images 'the product can be seen through', an impression can be obtained about the lateral glass thickness distribution. For instance, an image of the virtual product can be made in transparent view by combining the plurality of images into one image of the product in which a view of the product in transparent view is visible.

In particular, by the signal processor 14 the signals of the infrared camera 10, that is, the plurality of (in this example, six) successive images that have been made of the products 4.7-4.12 respectively, are processed in combination according to the principle of tomography, for obtaining a lateral glass thickness distribution of the virtual product 4'. To this end, the signal processing unit determines the rotational position of the products 4.7-4.12 from the images on the basis of the position of the markings M.i of the products 4.i. Thus, a virtual situation as represented in Figure 2 can be constructed by the signal processor 14. Because the camera 10 at the virtual positions indicated by cameras 10.7-10.12 makes recordings of the product 4 whereby through choice of the frequency sensitivity of the camera 10 'the product is seen through', this allows the principle of tomography to be applied in a manner known per se to reconstruct a 3D image of the virtual product 4'. This enables determining of the lateral glass thickness distribution of the product. The lateral glass thickness distribution can be expressed in numbers (for instance, a glass thickness at a particular position of the glass product) or by means of an image (for instance, an image of a view of the virtual glass product in transparent view where the glass thickness is also visible).

It holds, therefore, that by the signal processing unit the plurality of images with their associated determined orientations are processed in combination according to the principle of tomography for obtaining information about the lateral glass thickness distribution of the virtual glass product. In particular, it holds that the glass thickness distribution indicates relative variations in glass thickness. Also, it is possible that the glass thickness distribution comprises absolute values of the glass thickness distribution. Further, it is possible that the lateral glass thickness distribution is imaged on the display 18 in a 3D picture of the product 4'. Also, it is possible that a calibration measurement on a glass product having a known glass thickness is performed, on the basis of which thereupon step e. is carried out. For if the glass thickness is known upon the calibration measurement, the signal processing unit 14 can correct the lateral glass thickness distribution of the product 4'. Preferably, it holds that each image of the plurality of images both shows a side of the glass product that faces the sensor with which the image has been made and shows a side of the glass product, located opposite the side, that faces away from the sensor with which the image has been made.

Also, it holds preferably that in step e.'s processing in combination of the plurality of images according to the principle of tomography for obtaining the lateral glass thickness distribution of the glass of the virtual glass product, of the sensor, solely infrared light is used that relative to the sensor comes from the direction of an axial axis of the respective associated glass product.

Processing the images in combination according to the principle of tomography may be carried out according to a computation technique which is known by the name of "filter back projection", A.R.T. or S.A.R.T. In this way, the lateral glass distribution LGD of the virtual product has been determined as a function of h and phi. The glass distribution LGD (h,phi) can via a line 16 be shown on the display 18, for instance by means of numbers, a graph or an image. LGD is then for instance a number indicating the absolute wall thickness at the position (h,phi). It is also possible, however, that LGD indicates a relative wall thickness with respect to a reference wall thickness at the position (h, phi). Also, the lateral wall thickness may be depicted on the display in a 3D picture of the glass product. This picture then shows, for instance, a view of the glass product in transparent view. In this way, an operator can keep an eye on the glass thickness distribution of the successively produced products 4.i. When the glass thickness distribution starts to deviate, for instance because the wall thickness of the product becomes too large or too small at certain points, an intervention can be made in the production process, that is, an intervention can be made in the method step b. Such an intervention can be performed manually. However, it is also possible for such an intervention to be performed automatically with the aid of the signal processing unit, in this case via a feedback control line 30. It is possible, in case of a deviation of a glass thickness distribution, to correct manually or automatically in the production process of the products. It is also possible, in the case of a change per unit time in glass thickness distributions successively obtained spread in time and the change exceeding a predetermined value, to correct or control manually or automatically under control of the signal processing unit via line 30. Correcting or controlling may for instance be understood to mean adjusting a position and/or orientation of a trough and/or mould, providing a trough with a lubricant and/or replacing a mould. All this is schematically indicated in Figure 1.

It is noted that the aperture angle y of the camera 10 may also be smaller so that it has a value of for instance y' (see Figure 4). In that case, with the camera 10 a first area 26.1 is covered, the LGD of which is determined with the signal processing unit. In that case, using a second camera 10' which is also provided with an aperture angle y' and which is at a greater height h than the camera 10 and whose output signals are also supplied to the signal processing unit (not shown in the drawing), a lateral glass thickness distribution of the second area 26.2 that extends around the product 4' can be determined with the signal processing unit (see Fig. 4). The second area 26.2 is staggered in axial direction with respect to the area 26.1. Also, entirely analogously, it is possible that using a third camera (not shown) the lateral glass thickness distribution of an area 26.3 is determined. The areas 26.1, 26.2 and 26.3 then, for instance, adjoin each other. Accordingly, it holds for the areas 26.1 and 26.2 that steps d. and e. are carried out repeatedly for obtaining a lateral glass distribution LGD in a second area of the glass product that extends around an axial axis of the product, with the first and second area being staggered relative to each other in the axial direction.

In particular, it holds for the areas 26.1 to 26.3 that the steps d. and e., respectively, are carried out repeatedly at least three times for respectively obtaining lateral glass thickness distributions in respectively at least three mutually different areas which each extend around the axial axis and are staggered with respect to each other in axial direction and which preferably in combination cover, at least substantially, the whole glass product 4'.

Preferably, however, it holds that the aperture angle in vertical direction is so large that the respective area in which the glass thickness distribution is determined extends throughout the height of the product 4.i in the direction h. Further, it holds in this example that the aperture angle α of the camera 10 is such that each image shows the product, seen in its horizontal direction b, completely. However, the aperture angle may also be smaller or larger than shown. The images according to Figure 2 will, seen in horizontal direction, overlap each other partly so that different images show a same part of the product 4'. This is a minimal condition to be able to make use of the principle of tomography.

In this example, it holds that the infrared cameras are sensitive to light in the spectrum for which the glass is transparent, more particularly that each camera is sensitive to light having a bandwidth of 900nm - 3500nm, still more particularly that the sensor is sensitive to light having a bandwidth of 900nm - 1900nm.

If a trend in a deviation of successively determined lateral glass thickness distributions is determined, it may for instance be inferred that a particular part, such as a mould, is wearing.

The invention is not in any way limited to the embodiments outlined above. For instance, it is also possible that the camera 10 has an aperture angle y' while the camera 10, after making an image for obtaining a glass thickness distribution in the area 26.1, is moved up in axial direction for obtaining an image in order to obtain a glass thickness distribution in the area 26.2. After this, the camera may further be moved down for making an image of the area 26.3, etc. Further, it is clear that the camera 10 and the signal processing unit 14 can also be used in other production processes for forming glass products than described here. In fact, the camera in combination with the signal processing unit 14 constitutes an essential part of the invention. According to the invention, also, the cooling apparatus 7 could be omitted, since also without the cooling apparatus 7 the products will eventually cool down by themselves so that step c. may also be carried out without extra aids. Also, the cooling apparatus may, whether manually or automatically, be controlled (for example, the temperature of the cooling apparatus) on the basis of the determined LGD.

In particular, it holds that the infrared camera is a so-called high-speed infrared camera. However, other infrared cameras are also possible. In this example, a virtual product 4' has been constructed from recordings of the products 4.7-4.12. After this, for instance on the basis of the products 4.13-4.18 a next virtual product 4' may be constructed whose glass thickness distribution is determined entirely analogously to what has been discussed hereinabove for the products 4.7-4.12. More generally, from m recordings of the products 4.1+k with k=0, 1,2,3,...m-1, the glass thickness distribution of a virtual product can be obtained. Next, from m recordings of the products 4.1+m+k with k=0, 1,2,3,...m-1 a glass thickness distribution of a virtual product can be obtained. After this, from m recordings of the products 4.1+2m+k with k=0, 1,2,3,...m-1 a glass thickness distribution of a virtual product can be obtained and then from m recordings of the products 4.1+3m+k with k=0, 1,2,3,...m-1 a glass thickness distribution of a virtual product can be obtained. This can be continued so that after each m recordings a glass thickness distribution of a virtual product is determined. This brings with it that changes of the glass thickness distribution in time can be observed. This makes it possible to detect changes in the glass thickness distribution of successively determined virtual products as a result of for instance wear in troughs and the mould or changes in the glass thickness distribution of successive virtual products as a result of drifting settings of the trough and/or mould. When such a change exceeds a limiting value, automatically or manually the production flow may be controlled (as by setting a position and/or orientation of a trough and/or mould, supplying a lubricant to a trough, replacing a mould, etc.).

It is also possible that m is variable. In that case, the signal processing unit is configured to select a sufficient number of successive recordings in order that the virtual cameras associated with these recordings cover the associated virtual product completely around the axial axis of the virtual product. The signal processing unit then decides automatically which and how many recordings in each case are processed in combination for computing a glass thickness distribution of a virtual product.

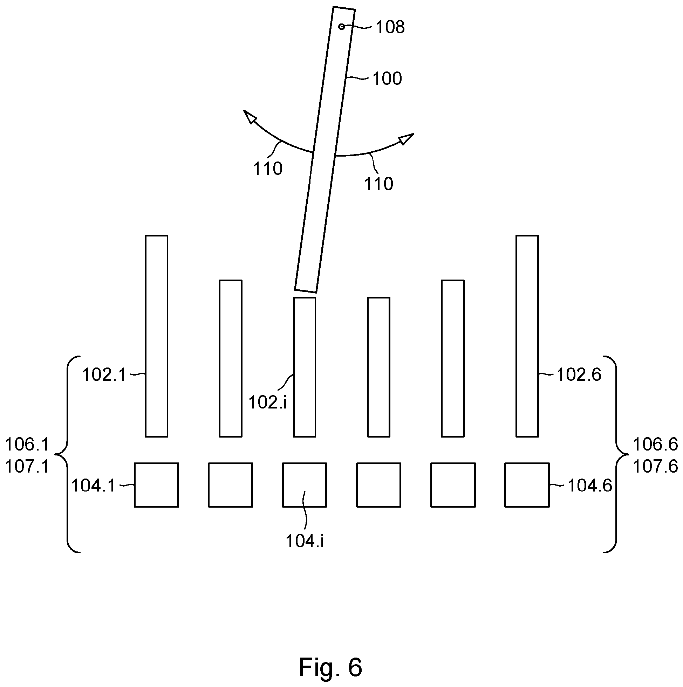

In an alternative embodiment, it is for instance possible that six products are formed parallel to each other in six parallel production flows. To this end, the system, in this example the product forming apparatus 3, includes six production flow paths 106.j which, for instance, each comprise at least one trough and a mould. This is shown in Figure 6 in which a glass gob formed in the heating apparatus 2 is guided via a switch 100 to a production flow path 106.j (j=1,2,3,..6) selected with the switch, each production flow path 106.j comprising at least one trough 102.j and a mould 104.j. The glass gobs that are successively transported along the production flow path 106.j together form a production flow 107.j. By rotation of the switch in the direction of arrows 110 around an axis (pivot) 108, other production flow paths 106.j can be selected with the switch. The glass gob flows, in this example, via the selected at least one trough 102.j to the mould 104.j. In this example, for j, successively the value 1,2,3,4,5,6 is selected by the switch 100. In this example, then, there are six parallel production flows.

Because there are six production flows, in succession six products 4.1, 4.2, 4.3, ...4.6 are formed which are placed in a row of six products on the conveyor. Here, product 4.1 has been formed from a glass gob which has been transported via production flow path 106.1 in production flow 107.1, product 4.2 has been formed from a glass gob which has been transported via production flow path 106.2 in production flow 107.2, product 4.3 has been formed from a glass gob which has been transported via production flow path 106.3 in production flow 107.3, etc. More generally, product 4.j has been formed from a glass gob which has been transported via production flow path 106.j in production flow 107.j, for j = 1,2,3,4,5,6. When thus six products have been produced, this process repeats itself.

Here, product 4.7 is formed from a glass gob which has been transported via production flow path 106.1 in production flow 107.1, product 4.8 is formed from a glass gob which has been transported via production flow path 106.2 in production flow 107.2, product 4.9 is formed from a glass gob which has been transported via production flow path 106.3 in production flow 107.3, etc. More generally, product 4.j+6 has been formed from a glass gob which has been transported via production flow path 106.j in production flow 107.j, for j = 1,2,3,4,5,6. When thus six products have been produced, this process repeats itself. Generally, therefore, it holds that product 4.j+n.6 with n=0,1,2,3,.... has been formed from a glass gob which has been transported via production flow path 106.j in production flow 107.j for j = 1,2,3,4,5,6.

The products 4.j, 4.j+6, 4.j+12, ....4.j+n.6 have then been made in a same production flow j, where j= 1,2,3,4,5,6 and n= 0,1,2,3,4,.....According to this variant, a number of successive recordings of products 4.j+n.6 for different values of n and a same value of j (and which thus belong to a same production flow j) are processed in combination with the signal processing unit for obtaining a glass thickness distribution of a virtual glass product. This number may for instance be eight. Thus, for a value of j, recordings of the products for n=0,1,2,..7 can be combined for obtaining a glass thickness distribution of a virtual product 4'. Next, for the same value of j, recordings of the products for n=8, 9, 10, ..15 can be processed in combination with the signal processing unit for obtaining a glass thickness distribution of another virtual product 4', etc. These recordings are obtained spread in time, and have been obtained of products that have been produced with the aid of the production flow path 106.j in the production flow 107.j. This makes it possible to detect changes in the glass thickness distribution of successive virtual products as a result of for instance wear in the at least one trough and/or mould of production flow path 106.j or changes in the glass thickness distribution of successive virtual products as a result of drifting settings in production flow path 106.j. When such a change exceeds a limiting value, automatically or manually settings of production flow path 106.j can be changed (such as a position and/or orientation of a trough and/or mould of the respective production flow path), a trough of production flow path 106.j can be lubricated, a mould of production flow path 106.j can be replaced, etc. It is thus possible, in case of a change in glass thickness distributions successively obtained spread in time and the change exceeding a predetermined value, to correct manually or automatically under control of the signal processing unit via line 30. It is also possible, in case of a deviation in a single glass thickness distribution and the deviation exceeding a predetermined value, to correct manually or automatically under control of the signal processing unit via line 30. Correcting may for instance be understood to mean adjusting a position and/or orientation of a trough and/or mould, providing a trough with a lubricant and/or replacing a mould.

All this can also be carried out for all other possible values of j so that glass thickness distributions are obtained for different virtual glass products relating to different production flow paths 106.j and hence to different production flows 107.j. If there is a deviation in a glass thickness distribution of a virtual product 4' that has been obtained from a production flow j, for instance the position of a trough and a mould belonging to the production flow path j may be corrected with respect to each other, or a trough belonging to the production flow path j may be provided with a lubricant. Also, a mould of the production flow path j. may be replaced. All of this may be carried out manually or automatically via line 30 under control of the signal processing unit. Also, changes in glass thickness distributions of virtual products that have successively been obtained with the aid of a same production flow path 106.j can be detected by the signal processing unit. If these changes exceed a predetermined value, again, as discussed above, settings of the respective production flow path 106.j may be adjusted, a trough of the respective production flow path 106.j may be lubricated and/or a mould of the respective production flow path may be replaced, manually or automatically.

In this example, in each case, eight images of products which have been obtained from a same production flow j were processed in combination for obtaining information about the glass thickness distribution of a virtual product. Instead of using in each case a= 8 images, the signal processing unit may also be configured to choose a to be variable, for instance such that a sufficient number of successive recordings are selected in order for the virtual cameras associated with these recordings to cover the associated virtual product completely around the axial axis of the virtual product. The signal processing unit then decides automatically which and how many recordings in each case are processed in combination for computing a glass thickness distribution of a virtual product associated with a particular production flow path j. Accordingly, it holds in particular that a plurality of the steps b. are carried out parallel to each other for producing parallel to each other a plurality of the products in a plurality of production flows which each comprise a step b., wherein each glass thickness distribution of a virtual product has been obtained on the basis of recordings of products that have been manufactured in a same production flow, more particularly wherein on the basis of at least one determined glass thickness distribution of a virtual product that has been obtained from recordings of products that stem from a same production flow, in an automatic manner the production flow is controlled, as by adjusting settings of that production flow.

Further, it holds thus, in particular, that the system is configured such that, in use, a plurality of the steps b. are carried out parallel to each other for producing parallel to each other a plurality of the products in a plurality of production flows which each comprise a step b., wherein the signal processing unit is configured such that, in use, each glass thickness distribution of a virtual product is obtained on the basis of recordings of products that have been manufactured in a same production flow, more particularly wherein on the basis of at least one determined glass thickness distribution of at least one virtual product that has been obtained from recordings of products that stem from a same production flow, in an automatic manner the production flow is controlled, as by adjusting settings of that production flow.

Finally, it is noted that automatic control loops via line 30 can comprise the adjusting, on the basis of a determined glass thickness distribution, of:
- Feeder temperature and temperature distribution
- Gob temperature distribution
- Gob forming process
- Gob loading process
- Mould cooling and residence time in the mould
- Design of the parison and the preform moulds
- Blowout process (B&B process)
- Plunger press process (NNPB, PB process)
- Plunger cooling process
- Blowout process at the front
- Shape and design of the blowing pipe
- Optimal lubricating method and lubricant moulds
- Optimal standing time determination of the moulds
- Optimal timing setting of the I.S. machine (glass forming machine)
- Optimal temperature settings of moulds and automatic control thereof
- Optimal air pressure control of product blowout
- Optimal air pressure control for making the parison
- Active reheating for an optimal temperature distribution of the glass gobs for an optimal LGD

Such variants each fall within the scope of the invention.

## Claims

1. Method for inspecting hollow glass products of glass product material, wherein said glass products are manufactured by:
a. heating the glass product material;
b. forming the heated glass product material into at least one glass product in a production flow;
c. cooling the formed glass product;
wherein inspecting the glass products comprises the following steps:
d. transporting the glass products formed in step b. successively along a predetermined path along at least one infrared light sensitive sensor, wherein with the at least one sensor, of a plurality of the glass products that are transported successively along the at least one sensor, with the at least one sensor per glass product an image is made, wherein step d. is carried out between steps b. and c.;
e. processing the images made in step d. for obtaining information about a wall thickness of the glass products, **characterised in that** the sensor is sensitive to infrared light having at least one frequency where a glass product of the plurality of glass products is transparent to the infrared light so that an image of the plurality of images both shows a side of the glass product that faces the sensor with which the image has been made and shows a side of the glass product, located opposite the side, that faces away from the sensor with which the image has been made, wherein
in step d. of each glass product of the plurality of glass products the rotational position of the glass product around an axial axis of the glass product relative to the at least one sensor is determined, wherein the images and the associated rotational positions of the plurality of products are processed in combination according to the principle of tomography for obtaining information about a lateral wall thickness distribution around an axial axis of a virtual glass product that represents an average of the plurality of glass products.

2. Method according to claim 1, **characterised in that** the glass thickness distribution indicates relative variations in glass thickness, and/or absolute values of the glass thickness distribution.

3. Method according to any one of the preceding claims, **characterised in that** a calibration measurement on a glass product having a known glass thickness is performed, on the basis of which thereupon step e. is carried out.

4. Method according to any one of the preceding claims, **characterised in that** each image of the plurality of images both shows a side of the glass product that faces the sensor with which the image has been made and shows a side of the glass product, located opposite the side, that faces away from the sensor with which the image has been made.

5. Method according to any one of the preceding claims, **characterised in that** in step e.'s processing in combination of the plurality of images according to the principle of tomography for obtaining the lateral glass thickness distribution of the virtual glass product, a) of the sensor solely infrared light is used that relative to the sensor comes from the direction of an axial axis of the respective associated glass product, and/or b) ray tracing is applied, and/or c) the processing in combination according to the principle of tomography comprises a computation technique according to (Filtered) Back Projection, A.R.T or S.A.R.T.

6. Method according to any one of the preceding claims, **characterised in that** in the steps d. and e. the lateral glass thickness distribution is determined in a first area of the virtual glass product that extends around the axial axis of the virtual glass product, and that that the steps d. and e.
are carried out repeatedly for obtaining a lateral glass thickness distribution in a second area of the virtual glass product that extends around the axial axis of the virtual glass product, wherein the first and second area are staggered with respect to each other in the axial direction;
wherein optionally the steps d. and e. are respectively carried out repeatedly at least three times for respectively obtaining lateral glass thickness distributions in respectively at least three mutually different areas which each extend around the axial axis of the virtual glass product and are staggered with respect to each other in axial direction and which preferably in combination cover at least substantially the whole virtual glass product, and/or
wherein optionally the sensor is displaced in the axial direction for obtaining recordings in the different areas.

7. Method according to any one of the preceding claims, **characterised in that** the glass product between steps b. and c. is transported along the path on a conveyor, such as from a product forming apparatus such as a mould in which the glass product has been formed in step b. to a cooling apparatus in which the product is cooled in step c, wherein optionally the rotational position of each of the glass products of the plurality of glass products around its axial axis on the conveyor is determined, e.g. with the aid of one of the sensors with which an image of the respective glass product is determined by recognizing of a predetermined marking (such as a seam or a dot) on or in the respective glass product.

8. Method according to any one of the preceding claims, **characterised in that** with the sensor an image is made of a glass product when an axial axis of the respective glass product is at least substantially intersected by an optical axis of the respective sensor.

9. Method according to any one of the preceding claims, **characterised in that** the sensor comprises an IR camera, in particular a high-speed IR camera, and/or the sensor is sensitive to light in the spectrum for which the glass product is transparent, more particularly that the sensor is sensitive to light having a bandwidth of 900nm - 3500nm, still more particularly that the sensor is sensitive to light having a bandwidth of 900nm - 1900nm.

10. Method according to any one of the preceding claims, **characterised in that** a sufficiently large number of recordings of products from a production flow are selected in order that these recordings in combination cover the associated virtual product completely around the axial axis of the virtual product.

11. Method according to any one of the preceding claims, **characterised in that** a plurality of the steps b. are carried out parallel to each other for producing parallel to each other a plurality of the products in a plurality of production flows which each comprise a step b., wherein each glass thickness distribution of a virtual product has been obtained on the basis of recordings of products that have been manufactured in a same production flow.

12. Method according to any one of the preceding claims, **characterised in that** in step d. the product is transported in a horizontally directed plane, wherein the axial axis of the product is vertically directed, and/or the rotational positions of different products can be different, more particularly that a plurality of products that are transported in step d. comprise mutually different rotational positions and/or that the glass products that are transported along the path mutually have a more or less random rotational position.

13. Method for producing and inspecting hollow glass products of glass product material, wherein said glass products are at least manufactured by:
a. heating the glass product material;
b. forming the heated glass product material into a glass product in a production flow;
c. cooling the formed glass product; wherein inspecting the glass products comprises the following steps:
d. transporting the glass products formed in step b. successively along a predetermined path along at least one infrared light sensitive sensor, wherein with the at least one sensor, of a plurality of the glass products that are transported successively along the at least one sensor, with the at least one sensor per glass product an image is made, wherein step d. is carried out between steps b. and c.;
e. processing the images made in step d. for obtaining information about a wall thickness of the glass products, **characterised in that** the method is further carried out according to the characterising portion of claim 1, optionally further **characterised by** one of claims 2-12.

14. System for inspecting glass products of glass product material according to the method according to the pre-characterising portion of claim 1, wherein the system comprises:
at least one sensor for carrying out step d.; and
a signal processing unit connected with the at least one sensor for processing signals coming from the at least one sensor, the signals each representing an image obtained with the at least one sensor, wherein the signal processing unit is configured for processing the plurality of images for obtaining information about a wall thickness of the glass products,
**characterised in that** the at least one sensor is sensitive to infrared light having at least one frequency where each glass product of the plurality of glass products is transparent to the infrared light so that an image of the plurality of images both shows a side of the glass product that faces the at least one sensor with which the image has been made and shows a side of the glass product, located opposite the side, that faces away from the at least one sensor with which the image has been made, wherein the system is configured such that, in use, in step d. of each glass product of the plurality of glass products the rotational position of the glass product around an axial axis of the glass product relative to the at least one sensor is determined, wherein the signal processing unit is configured such that, in use, the images and the associated rotational positions of the plurality of products are processed in combination according to the principle of tomography for obtaining information about a lateral wall thickness distribution around an axial axis of a virtual glass product that represents an average of the plurality of glass products.

15. System according to claim 14, **characterised in that** the system is further configured for carrying out a method according to the characterising portion of any one of claims 2-12.

16. System according to one of claims 14 or 15, **characterised in that** the system is configured such that, in use, a plurality of the steps b. are carried out parallel to each other for producing parallel to each other a plurality of the products in a plurality of production flows which each comprise a step b., wherein the signal processing unit is configured such that, in use, each glass thickness distribution of a virtual product is obtained on the basis of recordings of products that have been manufactured in a same production flow.

17. System according to claim 14, 15 or 16, wherein the system further comprises:
a heating apparatus for carrying out step a. of claim 1;
a product forming apparatus such as a mould for carrying out step b. of caim 1; and
a cooling apparatus for carrying out step c. of claim 1.

## Patentansprüche

1. Verfahren zur Prüfung von Hohlglasprodukten aus Glasproduktmaterial, wobei die besagten Glasprodukte hergestellt werden durch:
a. Erhitzen des Glasproduktmaterials;
b. Formung des erhitzten Glasproduktmaterials zu mindestens einem Glasprodukt in einem Fertigungsablauf;
c. Abkühlung des geformten Glasproduktes;
wobei die Prüfung der Glasprodukte die folgenden Schritte umfasst:
d. Transportieren der in Schritt b. gebildeten Glasprodukte nacheinander entlang eines vorbestimmten Weges entlang mindestens eines infrarotlichtempfindlichen Sensors, wobei mit dem mindestens einen Sensor von einer Mehrzahl der Glasprodukte, die nacheinander entlang des mindestens einen Sensors transportiert werden, mit dem mindestens einen Sensor pro Glasprodukt ein Bild gemacht wird, wobei Schritt d. zwischen den Schritten b. und c. durchgeführt wird;
e. Verarbeiten der in Schritt d. erstellten Bilder zur Erlangung von Informationen über eine Wanddicke der Glasprodukte, **dadurch gekennzeichnet, dass** der Sensor für Infrarotlicht empfindlich ist, mit mindestens einer Frequenz, bei welcher ein Glasprodukt der Mehrzahl von Glasprodukten für das Infrarotlicht transparent ist, so dass ein Bild der Mehrzahl von Bildern sowohl eine dem Sensor, mit dem das Bild erstellt wurde, zugewandte Seite des Glasprodukts zeigt, als auch eine der Seite gegenüberliegende Seite des Glasprodukts zeigt, die dem Sensor, mit dem das Bild erstellt wurde, abgewandt ist, wobei in Schritt d. von jedem Glasprodukt der Mehrzahl von Glasprodukten die Drehposition des Glasprodukts um eine axiale Achse des Glasprodukts relativ zu dem mindestens einen Sensor bestimmt wird, wobei die Bilder und die zugehörigen Drehpositionen der Mehrzahl von Produkten in Kombination nach dem Prinzip der Tomographie verarbeitet werden, zur Erlangung von Informationen über eine Seitenwanddickenverteilung um eine axiale Achse eines virtuellen Glasprodukts, das einen Durchschnitt der Mehrzahl von Glasprodukten darstellt.

2. Verfahren nach Anspruch 1, **dadurch gekennzeichnet, dass** die Glasdickenverteilung relative Schwankungen der Glasdicke und/oder absolute Werte der Glasdickenverteilung angibt.

3. Verfahren nach einem der vorhergehenden Ansprüche, **dadurch gekennzeichnet, dass** eine Kalibrierungsmessung an einem Glasprodukt mit einer bekannten Glasdicke durchgeführt wird, auf deren Grundlage daraufhin Schritt e. durchgeführt wird.

4. Verfahren nach einem der vorhergehenden Ansprüche, **dadurch gekennzeichnet, dass** jedes Bild der Mehrzahl von Bildern sowohl eine Seite des Glasproduktes zeigt, die dem Sensor, mit dem das Bild gemacht wurde, zugewandt ist, als auch eine der Seite gegenüberliegende Seite des Glasprodukte zeigt, die dem Sensor, mit dem das Bild gemacht wurde, abgewandt ist,

5. Verfahren nach einem der vorhergehenden Ansprüche, **dadurch gekennzeichnet, dass** im Schritt e. der Verarbeitung in nation der Mehrzahl der Bilder nach dem Prinzip der Tomographie zur Erlangung der seitlichen Glasdickenverteilung des virtuellen Glasproduktes a) vom Sensor ausschließlich Infrarotlicht verwendet wird, das relativ zum Sensor aus der Richtung einer Achslinie des jeweils zugehörigen Glasproduktes kommt, und/oder b) Raytracing angewendet wird, und/oder c) die Verarbeitung in Kombination nach dem Prinzip der Tomographie eine Berechnungstechnik gemäß (Gefilterte) Rückprojektion, A.R.T oder S.A.R.T. umfasst.

6. Verfahren nach einem der vorhergehenden Ansprüche, **dadurch gekennzeichnet, dass** in den Schritten d. und e. die seitliche Glasdickenverteilung in einem ersten Bereich des virtuellen Glasproduktes bestimmt wird, der sich um die axiale Achse des virtuellen Glasproduktes erstreckt, und dass die Schritte d. und e. wiederholt durchgeführt werden, um eine seitliche Glasdickenverteilung in einem zweiten Bereich des virtuellen Glasproduktes zu erhalten, der sich um die axiale Achse des virtuellen Glasproduktes erstreckt, wobei der erste und der zweite Bereich in axialer Richtung zueinander versetzt sind;
wobei optional die Schritte d. und e. jeweils mindestens dreimal wiederholt werden, um jeweils seitliche Glasdickenverteilungen in jeweils mindestens drei voneinander verschiedenen Bereichen zu erhalten, die sich jeweils um die axiale Achse des virtuellen Glasprodukts erstrecken und in axialer Richtung zueinander versetzt sind und die vorzugsweise in Kombination zumindest im Wesentlichen das gesamte virtuelle Glasprodukt abdecken, und/oder
wobei optional der Sensor in axialer Richtung verschoben wird, um Aufzeichnungen in den verschiedenen Bereichen zu erhalten.

7. Verfahren nach einem der vorhergehenden Ansprüche, **dadurch gekennzeichnet, dass** das Glasprodukt zwischen den Schritten b. und c. entlang des Weges auf einem Förderer transportiert wird, wie z.B. von einer Produktformungsvorrichtung, wie einer Form, in der das Glasprodukt in Schritt b. geformt wurde, zu einer Kühlvorrichtung, in der das Produkt in Schritt c. gekühlt wird, wobei optional die Drehposition jedes der Glasprodukte der Mehrzahl von Glasprodukten um seine axiale Achse auf dem Förderer bestimmt wird, z.B. mit Hilfe eines der Sensoren, mit dem ein Bild des jeweiligen Glasprodukts durch Erkennen einer vorbestimmten Markierung (wie einer Naht oder eines Punktes) auf oder in dem jeweiligen Glasprodukt bestimmt wird.

8. Verfahren nach einem der vorhergehenden Ansprüche, **dadurch gekennzeichnet, dass** mit dem Sensor ein Bild von einem Glasprodukt gemacht wird, wenn eine axiale Achse des jeweiligen Glasprodukts zumindest im Wesentlichen von einer optischen Achse des jeweiligen Sensors geschnitten wird.

9. Verfahren nach einem der vorhergehenden Ansprüche, **dadurch gekennzeichnet, dass** der Sensor eine IR-Kamera, insbesondere eine Hochgeschwindigkeits-IR-Kamera, umfasst und/oder der Sensor für Licht in dem Spektrum empfindlich ist, für welches das Glasprodukt transparent ist, insbesondere dass der Sensor für Licht mit einer Bandbreite von 900nm - 3500nm empfindlich ist, inbsensondere, dass der Sensor für Licht mit einer Bandbreite von 900nm - 1900nm empfindlich ist.

10. Verfahren nach einem der vorhergehenden Ansprüche, **dadurch gekennzeichnet, dass** eine ausreichend große Anzahl von Aufnahmen von Produkten aus einem Fertigungsablauf ausgewählt wird, so dass diese Aufnahmen in Kombination das zugehörige virtuelle Produkt vollständig um die axiale Achse des virtuellen Produkts abdecken.

11. Verfahren nach einem der vorhergehenden Ansprüche, **dadurch gekennzeichnet, dass** eine Mehrzahl der Schritte b. parallel zueinander durchgeführt werden, um parallel zueinander eine Mehrzahl der Produkte in einer Mehrzahl von Fertigungsabläufen herzustellen, die jeweils einen Schritt b. umfassen, wobei jede Glasdickenverteilung eines virtuellen Produkts auf der Grundlage von Aufzeichnungen von Produkten, die in einem gleichen Fertigungsablauf hergestellt wurden, erhalten wurde.

12. Verfahren nach einem der vorhergehenden Ansprüche, **dadurch gekennzeichnet, dass** in Schritt d. das Produkt in einer horizontal gerichteten Ebene transportiert wird, wobei die axiale Achse des Produktes vertikal ausgerichtet ist, und/oder die Drehpositionen verschiedener Produkte unterschiedlich sein können, insbesondere dass mehrere Produkte, die in Schritt d. transportiert werden, untereinander unterschiedliche Drehpositionen umfassen und/oder dass die Glasprodukte, die entlang des Weges transportiert werden, untereinander eine mehr oder weniger zufällige Drehposition aufweisen.

13. Verfahren zur Herstellung und Prüfung von Hohlglasprodukten aus Glasproduktmaterial, wobei die Glasprodukte mindestens hergestellt werden durch:
a. Erhitzen des Glasproduktmaterials;
b. Formung des erhitzten Glasproduktmaterials zu einem Glasprodukt in einem Fertigungsablauf;
c. Abkühlen des geformten Glasprodukts; wobei die Prüfung der Glasprodukte die folgenden Schritte umfasst:
d. Transportieren der in Schritt b. gebildeten Glasprodukte nacheinander entlang eines vorbestimmten Weges entlang mindestens eines infrarotlichtempfindlichen Sensors, wobei mit dem mindestens einen Sensor von einer Mehrzahl der Glasprodukte, die nacheinander entlang des mindestens einen Sensors transportiert werden, mit dem mindestens einen Sensor pro Glasprodukt ein Bild gemacht wird, wobei Schritt d. zwischen den Schritten b. und c. durchgeführt wird;
e. Verarbeiten der in Schritt d. erstellten Bilder zur Erlangung von Informationen über eine Wanddicke der Glasprodukte, **dadurch gekennzeichnet, dass** das Verfahren weiterhin gemäß dem charakterisierenden Abschnitt von Anspruch 1 durchgeführt wird, optional weiterhin **gekennzeichnet durch** einen der Ansprüche 2 bis 12.

14. System zur Prüfung von Glasprodukten aus Glasproduktmaterial gemäß dem Verfahren nach dem vor-charakterisierenden Abschnitt von Anspruch 1, wobei das System umfasst:
Mindestens einen Sensor zur Durchführung von Schritt d.; und
eine Signalverarbeitungseinheit, die mit dem mindestens einen Sensor verbunden ist, zum Verarbeiten von Signalen, die von dem mindestens einen Sensor kommen, wobei die Signale jeweils ein mit dem mindestens einen Sensor erhaltenes Bild darstellen, wobei die Signalverarbeitungseinheit zum Verarbeiten der Mehrzahl von Bildern konfiguriert ist, um Informationen über eine Wanddicke der Glasprodukte zu erhalten, **dadurch gekennzeichnet, dass** der mindestens eine Sensor für Infrarotlicht empfindlich ist, mit mindestens einer Frequenz, bei welcher jedes Glasprodukt der Mehrzahl von Glasprodukten für das Infrarotlicht transparent ist, so dass ein Bild der Mehrzahl von Bildern sowohl eine Seite des Glasprodukts zeigt, die dem mindestens einen Sensor, mit dem das Bild gemacht wurde, zugewandt ist, als auch eine der Seite gegenüberliegende Seite des Glasprodukts zeigt, die dem mindestens einen Sensor, mit dem das Bild gemacht wurde, abgewandt ist, wobei das System so konfiguriert ist, dass im Gebrauch im Schritt d. von jedem Glasprodukt der Mehrzahl von Glasprodukten die Drehposition des Glasprodukts um eine axiale Achse des Glasprodukts relativ zu dem mindestens einen Sensor bestimmt wird, wobei die Signalverarbeitungseinheit so konfiguriert ist, dass im Gebrauch die Bilder und die zugehörigen Drehpositionen der Mehrzahl von Produkten in Kombination nach dem Prinzip der Tomographie verarbeitet werden, zur Erlangung von Informationen über eine Seitenwanddickenverteilung um eine Achse eines virtuellen Glasprodukts, das einen Durchschnitt der Mehrzahl von Glasprodukten darstellt.

15. System nach Anspruch 14, **dadurch gekennzeichnet, dass** das System ferner so konfiguriert ist, dass es ein Verfahren gemäß dem charakterisierenden Abschnitt eines der Ansprüche 2 bis 12 durchführt.

16. System nach einem der Ansprüche 14 oder 15, **dadurch gekennzeichnet, dass** das System so konfiguriert ist, dass im Gebrauch eine Mehrzahl der Schritte b. parallel zueinander ausgeführt werden, um parallel zueinander eine Mehrzahl der Produkte in einer Mehrzahl von Fertigungsabläufen herzustellen, die jeweils einen Schritt b. umfassen, wobei die Signalverarbeitungseinheit so konfiguriert ist, dass im Gebrauch jede Glasdickenverteilung eines virtuellen Produkts auf der Grundlage von Aufzeichnungen von Produkten erhalten wird, die in einem gleichen Fertigungsablauf hergestellt worden sind.

17. System nach Anspruch 14, 15 oder 16, wobei das System ferner umfasst:
eine Heizvorrichtung zur Durchführung von Schritt a. nach Anspruch 1;
eine Produktformungsvorrichtung, wie z.B. eine Form, zur Durchführung des Schritts b. nach Anspruch 1; und
eine Kühlvorrichtung zur Durchführung des Schritts c. nach Anspruch 1.

## Revendications

1. Procédé d'inspection de produits en verre creux en matériau de produit en verre, dans lequel lesdits produits en verre sont fabriqués par :
a. chauffage du matériau du produit en verre ;
b. formage du matériau de produit en verre chauffé en au moins un produit en verre dans un flux de production ;
c. refroidissement du produit en verre formé ;
dans lequel l'inspection des produits en verre comprend les étapes consistant à :
d. transporter des produits en verre formés à l'étape b. successivement le long d'un trajet prédéterminé le long d'au moins un capteur sensible à la lumière infrarouge, dans lequel, avec le ou les capteurs, d'une pluralité de produits en verre qui sont transportés successivement le long du ou des capteurs, avec le ou les capteurs par produit en verre, une image est réalisée, dans lequel l'étape d. est effectuée entre les étapes b. et c. ;
e. traiter les images réalisées à l'étape d. pour obtenir des informations sur l'épaisseur de la paroi des produits en verre, **caractérisé en ce que** le capteur est sensible à la lumière infrarouge ayant au moins une fréquence à laquelle un produit en verre de la pluralité de produits en verre est transparent à la lumière infrarouge, de sorte qu'une image de la pluralité d'images montre à la fois un côté du produit en verre qui fait face au capteur avec lequel l'image a été réalisée et montre un côté du produit en verre, situé à l'opposé du côté, qui fait face au capteur avec lequel l'image a été réalisée, dans lequel, à l'étape d. de chaque produit en verre de la pluralité de produits en verre, la position de rotation du produit en verre autour d'un axe axial du produit en verre par rapport à l'au moins un capteur est déterminée, dans lequel les images et les positions de rotation associées de la pluralité de produits sont traitées en combinaison selon le principe de la tomographie pour obtenir des informations sur une répartition de l'épaisseur de la paroi latérale autour d'un axe axial d'un produit en verre virtuel qui représente une moyenne de la pluralité de produits en verre.

2. Procédé selon la revendication 1, **caractérisé en ce que** la répartition de l'épaisseur du verre indique des variations relatives de l'épaisseur du verre, et/ou des valeurs absolues de la répartition de l'épaisseur du verre.

3. Procédé selon l'une quelconque des revendications précédentes, **caractérisé en ce qu'**une mesure d'étalonnage est effectuée sur un produit en verre ayant une épaisseur de verre connue, sur la base de laquelle l'étape e. est ensuite effectuée.

4. Procédé selon l'une quelconque des revendications précédentes, **caractérisé en ce que** chaque image de la pluralité d'images montre à la fois un côté du produit en verre qui fait face au capteur avec lequel l'image a été réalisée et montre un côté du produit en verre, situé à l'opposé du côté, qui est tourné à l'opposé du capteur avec lequel l'image a été réalisée.

5. Procédé selon l'une quelconque des revendications précédentes, **caractérisé en ce que**, dans le traitement de l'étape e. en combinaison de la pluralité d'images selon le principe de la tomographie pour obtenir la répartition latérale de l'épaisseur du verre du produit en verre virtuel, a) du capteur, on utilise uniquement la lumière infrarouge qui, par rapport au capteur, provient de la direction d'un axe axial du produit en verre associé respectif, et/ou b) on applique le traçage de rayons, et/ou c) le traitement en combinaison selon le principe de la tomographie comprend une technique de calcul selon la rétroprojection (filtrée), A.R.T ou S.A.R.T.

6. Procédé selon l'une quelconque des revendications précédentes, **caractérisé en ce que**, dans les étapes d. et e., la répartition latérale de l'épaisseur du verre est déterminée dans une première zone du produit en verre virtuel qui s'étend autour de l'axe axial du produit en verre virtuel, et **en ce que** les étapes d. et e. sont effectuées de manière répétée pour obtenir une répartition latérale de l'épaisseur du verre dans une deuxième zone du produit en verre virtuel qui s'étend autour de l'axe axial du produit en verre virtuel, dans lequel les première et deuxième zones sont décalées l'une par rapport à l'autre dans la direction axiale ; dans lequel, éventuellement, les étapes d. et e. sont respectivement effectuées de manière répétée au moins trois fois pour obtenir respectivement des répartitions latérales de l'épaisseur du verre dans respectivement au moins trois zones mutuellement différentes qui s'étendent chacune autour de l'axe axial du produit en verre virtuel et sont décalées l'une par rapport à l'autre dans la direction axiale et qui, de préférence, en combinaison, couvrent au moins substantiellement l'ensemble du produit en verre virtuel, et/ou
dans lequel, éventuellement, le capteur est déplacé dans la direction axiale pour obtenir des enregistrements dans les différentes zones.

7. Procédé selon l'une quelconque des revendications précédentes, **caractérisé en ce que** le produit en verre entre les étapes b. et c. est transporté le long du trajet sur un convoyeur, par exemple d'un appareil de formation de produit tel qu'un moule dans lequel le produit en verre a été formé à l'étape b. à un appareil de refroidissement dans lequel le produit est refroidi à l'étape c, dans lequel, éventuellement, la position de rotation de chacun des produits en verre de la pluralité de produits en verre autour de son axe axial sur le convoyeur est déterminée, par exemple à l'aide de l'un des capteurs avec lesquels une image du produit en verre respectif est déterminée par la reconnaissance d'un marquage prédéterminé (tel qu'une soudure ou un point) sur ou dans le produit en verre respectif.

8. Procédé selon l'une quelconque des revendications précédentes, **caractérisé en ce qu'**avec le capteur, une image est faite d'un produit en verre lorsqu'un axe axial du produit en verre respectif est au moins sensiblement coupé par un axe optique du capteur respectif.

9. Procédé selon l'une quelconque des revendications précédentes, **caractérisé en ce que** le capteur comprend une caméra IR, en particulier une caméra IR à grande vitesse, et/ou le capteur est sensible à la lumière dans le spectre pour lequel le produit en verre est transparent, plus particulièrement **en ce que** le capteur est sensible à la lumière ayant une largeur de bande de 900 nm 3500 nm, encore plus particulièrement **en ce que** le capteur est sensible à la lumière ayant une largeur de bande de 900 nm 1900 nm.

10. Procédé selon l'une quelconque des revendications précédentes, **caractérisé en ce qu'**un nombre suffisamment important d'enregistrements de produits d'un flux de production est sélectionné afin que ces enregistrements en combinaison couvrent complètement le produit virtuel associé autour de l'axe axial du produit virtuel.

11. Procédé selon l'une quelconque des revendications précédentes, **caractérisé en ce qu'**une pluralité d'étapes b. sont réalisées parallèlement les unes aux autres pour produire parallèlement les unes aux autres une pluralité de produits dans une pluralité de flux de production qui comprennent chacun une étape b. , dans lequel chaque répartition d'épaisseur de verre d'un produit virtuel a été obtenue sur la base d'enregistrements de produits qui ont été fabriqués dans un même flux de production.

12. Procédé selon l'une quelconque des revendications précédentes, **caractérisé en ce qu'**à l'étape d. le produit est transporté dans un plan dirigé horizontalement, dans lequel l'axe axial du produit est dirigé verticalement, et/ou les positions de rotation de différents produits peuvent être différentes, plus particulièrement **en ce qu'**une pluralité de produits qui sont transportés à l'étape d. comprennent des positions de rotation mutuellement différentes et/ou **en ce que** les produits en verre qui sont transportés le long du trajet ont mutuellement une position de rotation plus ou moins aléatoire.

13. Procédé de production et d'inspection de produits en verre creux à partir d'un matériau de produit en verre, dans lequel lesdits produits en verre sont au moins fabriqués par :
a. le chauffage du matériau du produit en verre ;
b. le formage du matériau de produit en verre chauffé en un produit en verre dans un flux de production ;
c. le refroidissement du produit en verre formé ; dans lequel l'inspection des produits en verre comprend les étapes suivantes :
d. le transport des produits en verre formés à l'étape b. successivement le long d'un trajet prédéterminé le long d'au moins un capteur sensible à la lumière infrarouge, dans lequel, avec le ou les capteurs, d'une pluralité de produits en verre qui sont transportés successivement le long du ou des capteurs, avec le ou les capteurs par produit en verre, une image est réalisée, dans lequel l'étape d. est effectuée entre les étapes b. et c. ;
e. le traitement des images réalisées à l'étape d. pour obtenir des informations sur l'épaisseur de la paroi des produits en verre, **caractérisé en ce que** le procédé est en outre réalisé conformément à la partie caractérisante de la revendication 1, éventuellement en outre **caractérisé par** l'une des revendications 2 à 12.

14. Système d'inspection de produits en verre en matériau de produit en verre selon le procédé selon la partie pré-caractérisante de la revendication 1, dans lequel le système comprend :
au moins un capteur pour effectuer l'étape d. ; et
une unité de traitement de signaux connectée à l'au moins un capteur pour traiter les signaux provenant de l'au moins un capteur, les signaux représentant chacun une image obtenue avec l'au moins un capteur, dans lequel l'unité de traitement de signaux est configurée pour traiter la pluralité d'images afin d'obtenir des informations sur l'épaisseur de paroi des produits en verre, **caractérisé en ce que** le au moins un capteur est sensible à la lumière infrarouge ayant au moins une fréquence où chaque produit en verre de la pluralité de produits en verre est transparent à la lumière infrarouge, de sorte qu'une image de la pluralité d'images montre à la fois un côté du produit en verre qui fait face à l'au moins un capteur avec lequel l'image a été réalisée et montre un côté du produit en verre, situé à l'opposé du côté, qui fait face à l'opposé de l'au moins un capteur avec lequel l'image a été réalisée, dans lequel le système est configuré de telle sorte que, en cours d'utilisation, à l'étape d. de chaque produit en verre de la pluralité de produits en verre, la position de rotation du produit en verre autour d'un axe axial du produit en verre par rapport à l'au moins un capteur est déterminée, l'unité de traitement de signaux étant configurée de telle sorte que, en cours d'utilisation, les images et les positions de rotation associées de la pluralité de produits sont traitées en combinaison selon le principe de la tomographie pour obtenir des informations sur une répartition de l'épaisseur de la paroi latérale autour d'un axe axial d'un produit en verre virtuel qui représente une moyenne de la pluralité de produits en verre.

15. Système selon la revendication 14, **caractérisé en ce que** le système est en outre configuré pour mettre en œuvre un procédé selon la partie caractérisante de l'une quelconque des revendications 2 à 12.

16. Système selon l'une des revendications 14 ou 15, **caractérisé en ce que** le système est configuré de telle sorte que, en cours d'utilisation, une pluralité d'étapes b. sont réalisées parallèlement les unes aux autres pour produire parallèlement les unes aux autres une pluralité de produits dans une pluralité de flux de production qui comprennent chacun une étape b. , dans lequel l'unité de traitement de signaux est configurée de telle sorte que, en cours d'utilisation, chaque répartition d'épaisseur de verre d'un produit virtuel est obtenue sur la base d'enregistrements de produits qui ont été fabriqués dans un même flux de production.

17. Système selon la revendication 14, 15 ou 16, dans lequel le système comprend en outre :
un appareil de chauffage pour réaliser l'étape a. de la revendication 1 ;
un appareil de formation de produit tel qu'un moule pour réaliser l'étape b. de la revendication 1 ; et
un appareil de refroidissement pour réaliser l'étape c. de la revendication 1.
